# EUROPEAN PATENT APPLICATION

(11) **EP 4 549 583 A1**
(43) Date of publication of application: **07.05.2025**
(21) Application number: 23831897.6
(22) Date of filing: 28.06.2023
(51) Int. Cl.: C12Q 1/6848, C12Q 1/6851

(54) **METHOD FOR DETECTING TARGET RNA, INCLUDING TREATMENT WITH BLOCKER NUCLEIC ACID**

(30) Priority: 29.06.2022 KR 20220079602
(71) Applicant: Xenohelix. Co., Ltd., Incheon 21984 (KR)
(72) Inventor: CHO, Seok Keun, Incheon 21982 (KR); BYUN, Mi Young, Incheon 22000 (KR)
(74) Representative: Stolmár & Partner Patentanwälte PartG mbB
(86) International application number: PCT/KR2023/009024
(87) International publication number: WO 2024/005538

(57) **Abstract**

The present invention relates to a method for detecting a target RNA, which includes treating a blocker nucleic acid. In particular, the method of the present invention can inhibit non-specific reactions through the treatment of the blocker nucleic acid, and analyze even short RNA sequences, thereby enabling detection with high sensitivity and accuracy, and thus can be widely utilized for the diagnosis of various diseases such as infections and cancers.

## Description

### TECHNICAL FIELD

The present invention relates to a method for detecting target RNA including treatment with a blocker nucleic acid. In particular, the method of the present invention inhibits non-specific reactions through the treatment with a blocker nucleic acid and enables to analyze even RNA of short base sequences; therefore, the method enables to perform the detection with high sensitivity and accuracy, and thus can be widely used for the use of diagnosing various diseases, such as infectious diseases and cancer.

### BACKGROUND ART

As the quality of life improves, there is a growing interest in early diagnosis of diseases, and since molecular diagnostic technology directly detects the genetic information (DNA/RNA) of pathogens that cause diseases, it has been receiving much attention as a technology that can solve the shortcomings of immunodiagnostic technology that detects indirect factors of diseases based on existing antigen/antibody reactions.

In addition, with the recent outbreak of coronavirus disease-19 (COVID-19), there have been many deaths worldwide and the WHO has even declared a pandemic. In the case of these diseases caused by RNA viruses, the damage is greater due to high mutation rates, and early diagnosis of infection is more required.

Meanwhile, small RNAs such as miRNAs are protein-noncoding RNAs that exist *in vivo* and can act on the post-transcriptional process of a specific gene and regulate the expression of the corresponding gene. In particular, small RNAs are recognized as an important genetic element that mediates the maintenance of biological homeostasis by regulating biological functions, such as cell cycle, differentiation, development, metabolism, carcinogenesis, and aging, and particularly, their abnormal network formation may express fatal defects in cell physiology.

In addition, the expression pattern of small RNAs such as miRNA in the blood reacts sensitively in the early stages of cancer, and thus provides a strong advantage in early and predictive detection of cancer. Additionally, since a variety of cancers can be tested with a simple collection of blood, the burden on the patient's body can be reduced. Furthermore, in addition to the above-mentioned infections and cancers, there is a growing demand for the development of technology that enables early diagnosis by quickly detecting small RNA with high sensitivity in the diagnosis of various incurable diseases, such as Alzheimer's and Parkinson's diseases.

### [Prior Art Document]

(Patent Document 1) KR Patent Application Publication No. 10-2021-0086410
(Patent Document 2) KR Patent Application Publication No. 10-2021-0150041

### DISCLOSURE OF THE INVENTION

### TECHNICAL PROBLEM

An object of the present invention is to provide a method for detecting RNA,which includes treating with a blocker nucleic acid.

Another object of the present invention is a composition for detecting RNA, in which the composition includes a sensor DNA, which includes a target RNA recognition site and a module region; and a blocker nucleic acid including a sequence complementary to the module region of the sensor DNA.

### TECHNICAL SOLUTION

In order to achieve the above-described objects, an aspect of the present invention relates to a method for detecting RNA, in which the method includes: a) hybridizing a sensor DNA, which includes a sequence complementary to a target RNA to be detected, with the target RNA; b) performing polymerization using a polymerase, in which a module region of the sensor DNA is used as a template and the target RNA is used as a primer; and c) treating the blocker nucleic acid that complementarily binds to the sensor DNA thereto to inhibit amplification of the sensor DNA that does not hybridize with target RNA.

Specifically, the method may further include amplifying the polymerized strand formed through the polymerization step of the step b), through a PCR reaction simultaneously with the step c) or after the step c). In an embodiment of the present invention, it was confirmed that unbound sensor DNA was removed even when simultaneously treated with a blocker nucleic acid during qPCR. In this way, the blocker nucleic acid may be treated simultaneously during the PCR reaction, or may first be treated with a blocker nucleic acid and a PCR reaction may be performed thereafter, but the method is not limited thereto. Even if treated simultaneously during the PCR reaction, considering that unbound sensor DNA is easily removed, the treatment with a blocker nucleic acid before the PCR reaction would be able to sufficiently inhibit non-specific reactions even without performing the reaction for a long time.

Also specifically, the target RNA may be small RNA. The small RNA may collectively refer to RNA consisting of about 50 nucleotides or less in addition to miRNA and siRNA.

As used herein, the "blocker nucleic acid" refers to a nucleic acid molecule, which can complementarily bind to a sensor DNA including a recognition site for a target RNA, and bind to a sensor DNA, which has not reacted with the target RNA even after the hybridization of the target RNA and the sensor DNA, and inhibit the amplification of unreacted sensor DNA during a PCR amplification reaction. By removing non-specific signals other than the target RNA through such inhibition of the amplification of unreacted sensor DNA, it is possible to eliminate false positives and thereby show high accuracy.

Specifically, the blocker nucleic acid may include a sequence complementary to the module region in the sensor DNA, but is not limited thereto, and if necessary, the region to which the blocker nucleic acid binds may be changed within the region of the sensor DNA to thereby provide a sequence complementary thereto.

Also specifically, the blocker nucleic acid may complementarily bind to the module region in the sensor DNA, but is not limited thereto, and if necessary, may complementarily bind to the sensor DNA region where binding of the blocker nucleic acid is required.

Also specifically, the blocker nucleic acid may include locked nucleic acid (LNA).

The locked nucleic acid may be included in a ratio of 5% to 50% in the blocker nucleic acid, more specifically, the locked nucleic acid may be included in a ratio of 15% to 50% in the blocker nucleic acid, and most specifically, the locked nucleic acid may be included in a ratio of 30% to 50% in the blocker nucleic acid.

Additionally, the position of the nucleotide in the blocker nucleic acid to be substituted with the lock nucleic acid is not limited.

Additionally, the sensor DNA may be in the form of single strand (ss) DNA.

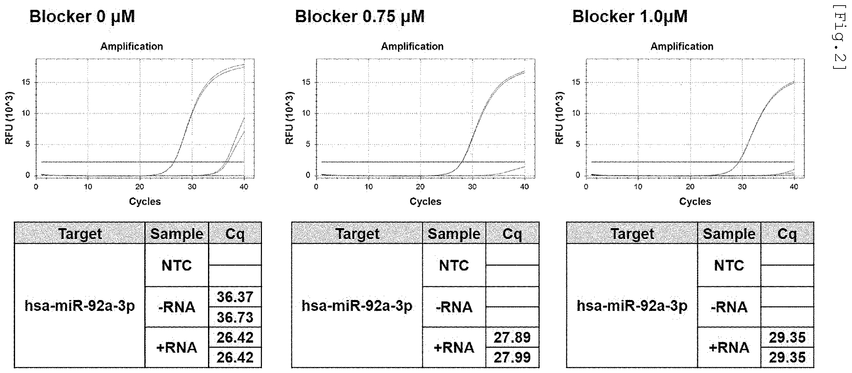

In an embodiment of the present invention, a reaction for the detection of target RNA was performed by mixing, to a sample, a sensor DNA, which was in the form of ssDNA and included a sequence complementary to the target RNA, and it was confirmed that when a blocker nucleic acid was treated in the detection reaction above, unreacted sensor DNA which did not hybridize with the target RNA was effectively removed thereby inhibiting a non-specific PCR reaction (FIGS. 2 to 5).

Another aspect of the invention relates to a composition for detecting RNA, which includes a sensor DNA that includes a target RNA recognition site and a module region; and a blocker nucleic acid which includes a sequence complementary to the module region of the sensor DNA.

Specifically, the target RNA recognition site of the sensor DNA may include a sequence complementary to the target RNA, which is a subjectto be detected.

Also specifically, the blocker nucleic acid may include locked nucleic acid (LNA). Blocker nucleic acids are as described above.

The composition for detecting RNA may further include a buffer solution, PCR primers, etc., In addition to sensor DNA; and a blocker nucleic acid, and the additionally-included components may be changed and applied as needed.

### ADVANTAGEOUS EFFECTS

The RNA detection method of the present invention and the constitution of the sensor DNA used for the detection have a very low detection limit at the femtomole (fmol) and attomole (amol) levels, and thus are significantly superior in sensitivity and accuracy compared to conventional RNA detection technologies. In particular, the accuracy of detection may be further improved by inhibiting non-specific reactions by treating with a blocker nucleic acid.

The effects of the present invention are not limited to those effects described above, and should be understood to include all effects that can be derived from the constitution of the invention described in the detailed description or claims of the present invention.

### BRIEF DESCRIPTION OF THE DRAWINGS

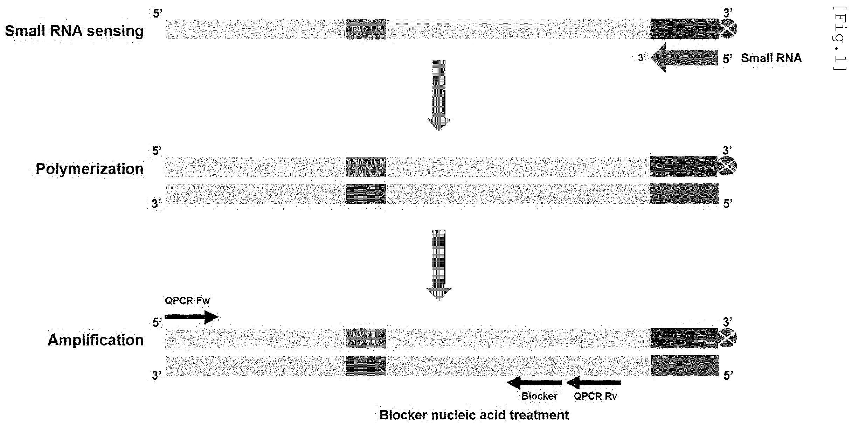

FIG. 1 shows a schematic diagram of the small RNA detection method of the present invention, which includes the treatment with a blocker nucleic acid.
FIG. 2 shows the results confirming the effect of removing unbound sensor DNA upon treatment with the blocker nucleic acid of the present invention.
FIG. 3 shows the results of confirming the effect of removing unbound sensor DNA depending on the LNA content in the blocker nucleic acid.
FIG. 4 shows the results of confirming the effect of removing unbound sensor DNA depending on the LNA position in the blocker nucleic acid.
FIG. 5 shows the results of confirming the effect of removing unbound sensor DNA of the blocker nucleic acid depending on the type of sensor DNA.

### MODE FOR CARRYING OUT THE INVENTION

Hereinafter, the present invention will be described in detail by examples. However, the following examples are merely illustrative of the present invention, and the present invention is not limited by the following examples.

### Preparation Example 1. Design of blocker nucleic acid

A blocker nucleic acid capable of complementarily binding to sensor DNA including a recognition site for target RNA was designed. The sensor DNA includes a recognition site including a sequence complementary to the target RNA, and the recognition site detects the target RNA and hybridizes with the target RNA and was named 'sensor DNA'.

Since the sensor DNA that has not hybridized with the target RNA may remain as ssDNA and cause a non-specific reaction in detection, a step to remove the sensor DNA is required before performing a PCR reaction for detection. In the present invention, a blocker nucleic acid consisting of a sequence complementary to the sensor DNA was designed, and it was confirmed that the treatment with the blocker nucleic acid can inhibit non-specific reactions to be caused by sensor DNA that has not hybridized with the target RNA. The blocker nucleic acid was designed to include locked nucleic acid (LNA).

The sequence of the blocker nucleic acid exemplarily designed in the present invention is shown in Table 1 below. The blocker nucleic acid sequence may be modified and applied as needed.

**[Table 1]**

| SEQ ID NO: | Category | Sequence of (5'-3') |
|---|---|---|
| 1 | blocker nucleic acid | TGAGTGGGCACTCCGCTGCC |

### Experimental Example 1. Confirmation of effect of removing unbound sensor DNA according to treatment with blocker nucleic acid

RNA was purified from human blood samples using the XENOPURE Blood Small RNA Purification Kit (XENOHELIX). After mixing 100 ng of whole blood RNA and 2 fmol of hsa-miR-92a-3p sensor DNA, 2 µL of reaction buffer (200 mM Tris-HCl, 100 mM (NH₄)₂SO₄, 100 mM KCl, 20 mM MgSO₄, 1% Triton X-100(pH 8.8 at 25°C)), 1 µL of 2.5 mM dNTP, and 2 units of DNA polymerase (XenoT-POL) were mixed, and the mixed solution was heated at 95°C for 3 minutes and then incubated at 63°C for 5 minutes to synthesize DNA complementary to the hsa-miR-92a-3p sensor DNA. As a negative control (NTC), distilled water was added in place of RNA and a reaction was performed in the same manner.

Thereafter, non-specific binding was broken by treating with nuclease at 60°C for 10 minutes, and the sensor, which was ssDNA, was removed, and the remaining sensor and RNA were further removed by passing through a column.

3 µL each of the samples was subjected to qPCR using hsa-miR-92a-3p sensor-specific primers under the conditions of 1 cycle of 95°C for 10 minutes, and 40 cycles of 95°C for 15 seconds and 60°C for 1 minute, in which the blocker nucleic acid was treated at concentrations of 0 µM, 0.75 µM, and 1.0 µM in performing the qPCR.

As a result, as shown in FIG. 2, it was confirmed that when the blocker nucleic acid was treated, all sensor DNA unbound to the target RNA was removed and no PCR results were obtained (-RNA: sensor DNA (ssDNA) unbound to the target RNA; +RNA: sensor DNA bound to the target RNA (formation of a double strand)).

From the results above, it was confirmed that in the RNA detection technology of the present invention, which includes a step of treating with a blocker nucleic acid, the sensor DNA in the form of ssDNA used as a detection means is removed when it fails to hybridize with the target RNA, and thus, a non-specific detection reaction does not occur.

### Experimental Example 2. Confirmation of effect of removing unbound sensor DNA according to LNA content in blocker nucleic acid

The effect of removing ssDNA sensor depending on the LNA content in the blocker nucleic acid was confirmed. For the blocker nucleic acid sequences shown in Table 1, blocker nucleic acids were prepared by varying the content of LNA as shown below, and the portions substituted with LNA are underlined and indicated in bold.

**[Table 2]**

| Category | LNA | LNA Position (underlined and indicated in bold) |
|---|---|---|
| Example 1 | LNA 2 mer x3 | 5' - **TG**AGTGGGC**AC**TCCGCTG**CC** - 3' |
| Example 2 | LNA 3 mer x 3 | 5' - **TGA**GTGGG**CAC**TCCGCT**GCC** - 3' |

RNA was obtained from human blood samples in the same manner as in Example 1 above, and 100 ng of whole blood RNA and 2 fmol of hsa-miR-92a-3p sensor DNA were mixed and then mixed with 2 µL of reaction buffer (200 mM Tris-HCl, 100 mM (NH₄)₂SO₄, 100 mM KCl, 20 mM MgSO₄, 1% Triton X-100(pH 8.8 at 25°C)), 1 µL of 2.5 mM dNTP, and 2 units of DNA polymerase (XenoT-POL) were mixed. The mixed solution was heated at 95°C for 3 minutes and then incubated at 63°C for 5 minutes to synthesize DNA complementary to the hsa-miR-92a-3p sensor DNA. As a negative control (NTC), distilled water was added in place of RNA and a reaction was performed in the same manner.

Thereafter, non-specific binding was broken by treating with nuclease at 60°C for 10 minutes, and the sensor, which was ssDNA, was removed, and the remaining sensor and RNA were further removed by passing through a column.

3 µL each of the samples was subjected to qPCR using hsa-miR-92a-3p sensor-specific primers under the conditions of 1 cycle of 95°C for 10 minutes, and 40 cycles of 95°C for 15 seconds and 60°C for 1 minute, in which the blocker nucleic acids of Example 1, and Example 2 were each treated at a concentration of 0.75 µM.

The Cq values of the negative control group, in which the blocker nucleic acid was not added, and the treatment groups (*i.e*., Example 1 and Example 2), in which the blocker nucleic acid was added, were compared.

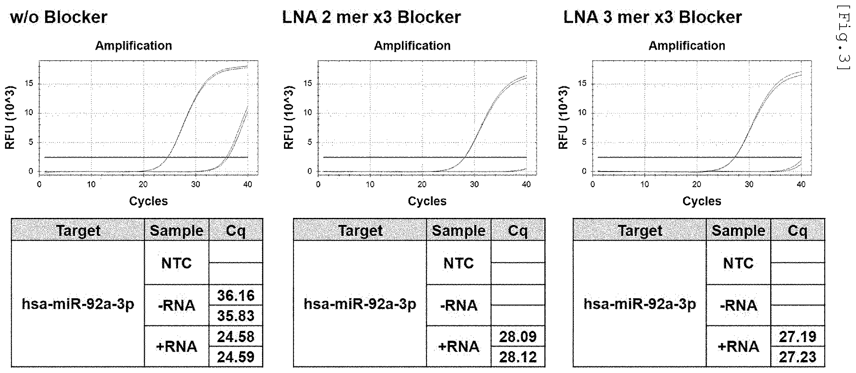

As a result, as shown in FIG. 3, it was confirmed that when the blocker nucleic acids were treated as in Examples 1 and 2, all sensor DNA unbound to the target RNA was removed and no PCR results were obtained (-RNA: sensor DNA (ssDNA) unbound to the target RNA; +RNA: sensor DNA bound to the target RNA (formation of a double strand). From these results, it was confirmed that ssDNA sensor-specific removal can be performed stably even if the content of LNA in the blocker nucleic acid changes.

### Experimental Example 3. Confirmation of effect of removing unbound sensor DNA according to LNA position in blocker nucleic acid

The effect of ssDNA sensor removal depending on the LNA content in the blocker nucleic acid was confirmed. For the blocker nucleic acid sequence shown in Table 1, blocker nucleic acids were prepared by substituting 9 nucleotides with LNA and varying the positions of the substituted LNA as shown below, and the portions substituted with LNA are underlined and indicated in bold.

**[Table 3]**

| Example | LNA | LNA Position (underlined and indicated in bold) |
|---|---|---|
| Example 2 | LNA 3 mer x 3 | 5' - **TGA**GTGGG**CAC**TCCGCT**GCC** - 3' |
| Example 3 | LNA 5 mer + 4 mer | 5' - **TGAGT**GGGCACTCCGC**TGCC** - 3' |
| Example 4 | LNA 6 mer + 3 mer | 5' - **TGAGTG**GGCACTCCGCT**GCC** - 3' |
| Example 5 | LNA 9 mer | 5' - **TGAGTGGGC**ACTCCGCTGCC - 3' |

RNA was obtained from human blood samples in the same manner as in Example 1 above, and 100 ng of whole blood RNA and 2 fmol of hsa-miR-92a-3p sensor DNA were mixed and then mixed with 2 µL of reaction buffer (200 mM Tris-HCl, 100 mM (NH₄)₂SO₄, 100 mM KCl, 20 mM MgSO₄, 1% Triton X-100(pH 8.8 at 25°C)), 1 µL of 2.5 mM dNTP, and 2 units of DNA polymerase (XenoT-POL) were mixed. The mixed solution was heated at 95°C for 3 minutes and then incubated at 63°C for 5 minutes to synthesize DNA complementary to the hsa-miR-92a-3p sensor DNA. As a negative control (NTC), distilled water was added instead of RNA and a reaction was performed in the same manner.

Thereafter, non-specific binding was broken by treating with nuclease at 60°C for 10 minutes, and the sensor, which was ssDNA, was removed, and the remaining sensor and RNA were further removed by passing through a column.

3 µL each of the samples was subjected to qPCR using hsa-miR-92a-3p sensor-specific primers under the conditions of 1 cycle of 95°C for 10 minutes, and 40 cycles of 95°C for 15 seconds and 60°C for 1 minute, in which the blocker nucleic acids of Example 2 to Example 5 were each treated at a concentration of 0.75 µM.

The Cq values of the negative control group, in which the blocker nucleic acid was not added, and the treatment groups (i.e., Example 2 to Example 5), in which the blocker nucleic acid was added, were compared.

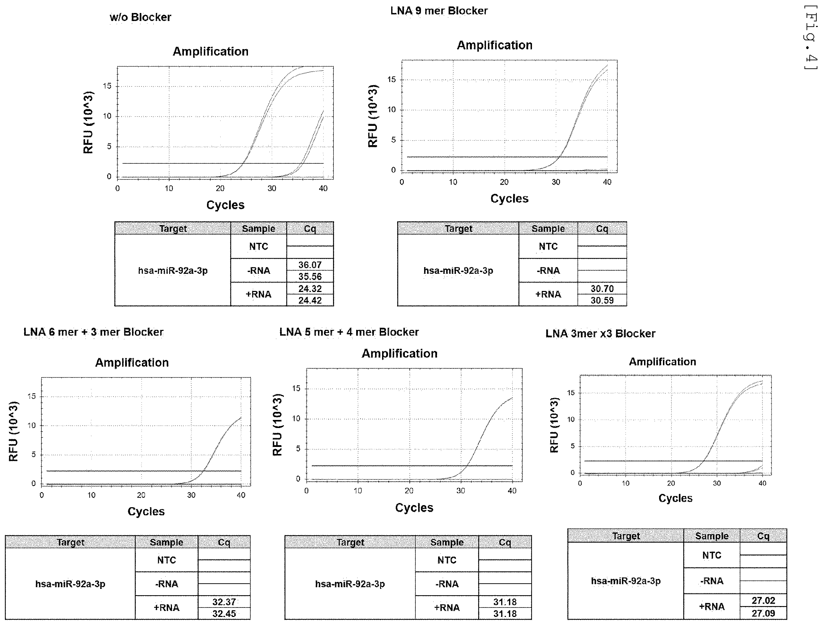

As a result, as shown in FIG. 4, it was confirmed that in all cases where the blocker nucleic acids of Example 2 to Example 5 were treated, all sensor DNA unbound to the target RNA was removed and no PCR results were obtained (-RNA: sensor DNA (ssDNA) unbound to the target RNA; +RNA: sensor DNA bound to the target RNA (formation of a double strand). From these results, it was confirmed that ssDNA sensor-specific removal can be performed stably even if the position of LNA in the blocker nucleic acid changes.

### Experimental Example 4. Confirmation of effect of removing unbound sensor DNA of blocker nucleic acid according to type of sensor DNA

The effect of blocker nucleic acids on ssDNA sensor removal was confirmed for different types of sensor DNA.

RNA was obtained from human blood samples in the same manner as in Example 1 above, and 100 ng of whole blood RNA and 2 fmol each of hsa-miR-92a-3p, hsa-miR-144-3p, and hsa-let-7d-5p sensor DNA were mixed and then mixed with 2 µL of reaction buffer (200 mM Tris-HCl, 100 mM (NH₄)₂SO₄, 100 mM KCl, 20 mM MgSO₄, 1% Triton X-100(pH 8.8 at 25°C)), 1 µL of 2.5 mM dNTP, and 2 units of DNA polymerase (XenoT-POL) were mixed. The mixed solution was heated at 95°C for 3 minutes and then incubated at 63°C for 5 minutes to synthesize DNA complementary to the hsa-miR-92a-3p sensor DNA. As a negative control (NTC), distilled water was added instead of RNA and a reaction was performed in the same manner.

Thereafter, non-specific binding was broken by treating with nuclease at 60°C for 10 minutes, and the sensor, which was ssDNA, was removed, and the remaining sensor and RNA were further removed by passing through a column.

3 µL each of the samples was subjected to qPCR using hsa-miR-92a-3p sensor-specific primers under the conditions of 1 cycle of 95°C for 10 minutes, and 40 cycles of 95°C for 15 seconds and 60°C for 1 minute, in which the blocker nucleic acids of Example 2 were each treated at a concentration of 0.75 µM.

The Cq values of the negative control group, in which the blocker nucleic acid was not added, and the treatment groups, in which the blocker nucleic acid was treated while varying the type of each sensor, were compared.

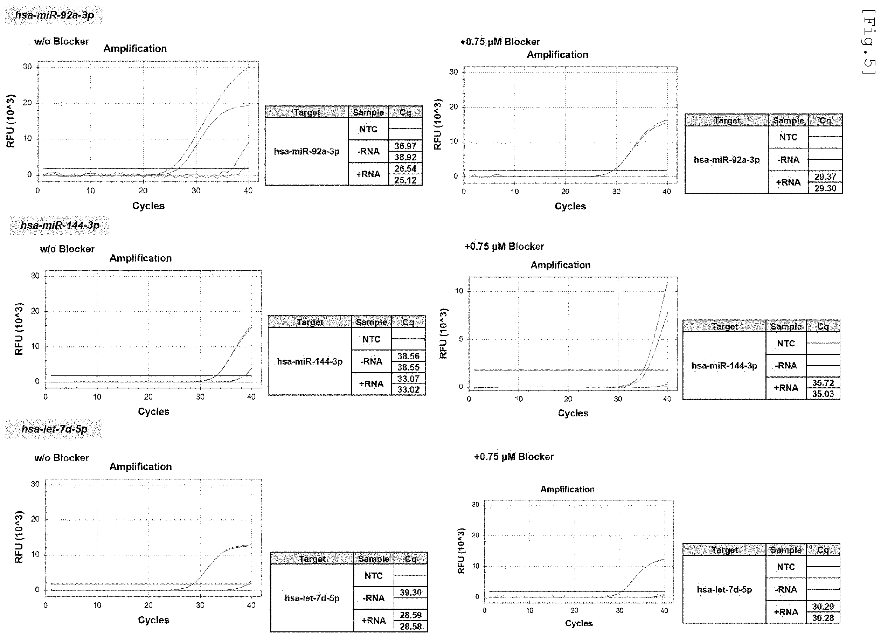

As a result, as shown in FIG. 5, it was confirmed that regardless of the type of sensor, the sensor was not bound to the target RNA in all of the hsa-miR-92a-3p, hsa-miR-144-3p, and hsa-let-7d-5p sensor DNA groups and thus no PCR results were obtained (-RNA: sensor DNA (ssDNA) unbound to the target RNA; +RNA: sensor DNA bound to the target RNA (formation of a double strand). From these results, it was confirmed that ssDNA sensor-specific removal can be performed stably by the treatment with a blocker nucleic acid, regardless of the type of sensor.

That is, the RNA detection method of the present invention, which includes the step of treating with a blocker nucleic acid, can significantly improve detection sensitivity by eliminating non-specific reactions, and non-specific reactions can be controlled even if the content or location of LNA included in the blocker nucleic acid varies and regardless of the type of sensor DNA.

The sensor DNA sequences for detecting each miRNA used in Experimental Examples 1 to 4 are summarized in Table 4 below. The portions within the sensor DNA to which the blocker nucleic acid binds are underlined.

**[Table 4]**

| SEQ ID NO: | Sensor DNA (ssDNA) | Sequence (5'-3') |
|---|---|---|
| 2 | hsa-miR-92a-3p Sensor DNA | AACAATACCACGACCACCGACTGAAAGGGTGGATCGTGAAGG GGGCTTCTTGTTGGACAGTCAGTGGATGCTCAAGGCCAGGGC AGCGGAGTGCCCACTCAGATCTGCTCCTTGTGCTTCAGTTGC TTGGCTCTGGTGTATTGGTACAGGCCGGGACAAGTGCAATA |
| 3 | hsa-miR-144-3p Sensor DNA | AACAATACCACGACCACCGACGTGCTTGGAGTCAATCGGATG TAGGATGGTCTCCAGACACCGGACCACTCTTCACGCCTAAAG CATAAACGTCGAGCAGTGGCAGCGGAGTGCCCACTCAGAAAG TCTTAGTACCGGACGTGCCGTTTCACTGCGGTTGCTTGGCTC TGGTGTATTGGTAGTACATCATCTATACTGTA |
| 4 | hsa-let-7d-5p Sensor DNA | AACAATACCACGACCACCGACGAGGGCACAATCCTATTCCCA TTTGTATGTTCAGCTAACTTCTACCCATCCCCGGCAGCGGAG TGCCCACTCAGTTAAGTAGGTCGTGAGATGCCATGGAGGCTC TCGTTCATCCCGTGGGTTGCTTGGCTCTGGTGTATTGGT AACTATGCAACCTACTACCTCT |

In addition, the primer sequences used when performing qPCR are summarized in Table 5 below.

**[Table 5]**

| SEQ ID NO: | Target RNA | Type | Sequence (5'-3') |
|---|---|---|---|
| 5 | hsa-miR-92a-3p | forward primer | TGAAAGGGTGGATCGTGAAG |
| 6 | hsa-miR-92a-3p | reverse primer | TACCAATACACCAGAGCCAAG |
| 7 | hsa-miR-92a-3p | probe | AGTCAGTGGATGCTCAAGGCCAG |
| 8 | hsa-miR-144-3p | forward primer | CTTGGAGTCAATCGGATGTAGG |
| 9 | hsa-miR-144-3p | reverse primer | CACGTCCGGTACTAAGACTTTC |
| 10 | hsa-miR-144-3p | probe | CGCCTAAAGCATAAACGTCGAGCAGT |
| 11 | hsa-let-7d-5p | forward primer | GGCACAATCCTATTCCCATTTG |
| 12 | hsa-let-7d-5p | reverse primer | GGCATCTCACGACCTACTTAAC |
| 13 | hsa-let-7d-5p | probe | ATGTTCAGCTAACTTCTACCCATCCCC |

The above description of the present invention is for illustrative purposes, and those of ordinary skill in the art to which the present invention pertains will understand that it can easily be modified into other specific forms without changing the technical idea or essential features of the present invention. Therefore, it should be understood that the examples described above are illustrative in all respects and not restrictive. For example, each component described as a single entity may also be implemented in a distributed manner, and likewise, components described as distributed may be implemented in a combined manner.

The scope of the present invention is indicated by the claims set forth below, and all changes or modifications derived from the meaning and scope of the claims and their equivalent concepts should be interpreted as being included in the scope of the present invention.

## Claims

1. A method for detecting RNA, the method comprising:
a) hybridizing a sensor DNA, which comprises a sequence complementary to a target RNA to be detected, with the target RNA;
b) performing polymerization using a polymerase, in which a module region of the sensor DNA is used as a template and the target RNA is used as a primer; and
c) treating the blocker nucleic acid that complementarily binds to the sensor DNA thereto to inhibit amplification of the sensor DNA that does not hybridize with target RNA.

2. The method of claim 1, further comprising amplifying a polymerized strand, which is formed through the polymerization of the step b), through a PCR reaction simultaneously with the step c) or after the step c).

3. The method of claim 1, wherein the target RNA is a small RNA.

4. The method of claim 3, wherein the small RNA is an miRNA.

5. The method of claim 1, wherein the blocker nucleic acid comprises a sequence complementary to the module region within the sensor DNA.

6. The method of claim 1, wherein the blocker nucleic acid complementarily binds to the module region within the sensor DNA.

7. The method of claim 1, wherein the blocker nucleic acid comprises a locked nucleic acid (LNA).

8. The method of claim 5, wherein the locked nucleic acid (LNA) is included in a proportion of 5% to 50% in the blocker nucleic acid.

9. The method of claim 1, wherein the sensor DNA is in the form of single strand (ss) DNA.

10. A composition for detecting RNA, the composition comprising:
a sensor DNA, which comprises a target RNA recognition site and a module region; and
a blocker nucleic acid comprising a sequence complementary to the module region of the sensor DNA.

11. The method of claim 10, wherein the target RNA recognition site of the sensor DNA comprises a sequence complementary to the target RNA to be detected.

12. The method of claim 10, wherein the blocker nucleic acid comprises a locked nucleic acid (LNA).
